# EUROPEAN PATENT SPECIFICATION

(11) **EP 3 911 244 B1**
(45) Date of publication and mention of the grant of the patent: **18.02.2026**
(21) Application number: 20704999.0
(22) Date of filing: 16.01.2020
(51) Int. Cl.: A61B 8/08, A61B 8/12, A61C 9/00, A61B 8/00

(54) **METHOD OF DETECTING AN ABNORMALITY ALONG A PORTION OF A DENTAL ARCH**
VERFAHREN ZUR DETEKTION EINER ANOMALIE ENTLANG EINES ABSCHNITTS EINES ZAHNBOGENS
PROCÉDÉ DE DÉTECTION D'UNE ANOMALIE LE LONG D'UNE PARTIE D'UNE ARCADE DENTAIRE

(30) Priority: 16.01.2019 US 201962793357 P
(43) Date of publication of application: 24.11.2021
(73) Proprietor: Trophy, 77435 Marne La Vallée Cedex 2 (FR); Carestream Dental LLC, Atlanta, GA 30339 (US)
(72) Inventor: CAPRI, Arnaud, 77435 Marne La Vallée Cedex 2 (FR); ROUDERGUES, David, 77435 Marne La Vallée Cedex 2 (FR); INGLESE, Jean-Marc, 77435 Marne La Vallée Cedex 2 (FR); SHELLARD, Edward, Atlanta, GA 30339 (US); JOSSO, Hervé, 77435 Marne La Vallée Cedex 2 (FR)
(74) Representative: Casalonga
(86) International application number: PCT/EP2020/051062
(87) International publication number: WO 2020/148406

(56) References cited:
- WO-A1-2018/055747
- JP-A- S61 115 547
- US-A- 5 100 318
- US-A- 5 755 571
- US-A1- 2009 306 506
- US-A1- 2015 105 665
- US-A1- 2017 119 505
- US-A1- 2018 168 781

## Description

### FIELD OF THE INVENTION

The present invention relates to the technical field of ultrasonic probes and of ultrasonic dental probes for screening a region of interest, such as a periodontium or a gingiva area of a patient mouth.

### BACKGROUND OF THE INVENTION

Ultrasound imaging has been adapted for intraoral use in a number of implementations and has been found to have particular utility for tasks such as measurement of periodontal pocket depth. Periodontal diseases such as gingivitis, for example, can be detected by sensing the acoustic response of tissues. Ultrasound may also provide accurate information about the pathological nature of lesions.

By comparison with conventional methods used for periodontal pocket evaluation, ultrasound probing is relatively more comfortable and can be more accurate. Because it uses only ultrasound signals, ultrasound probing is inherently safer than methods that employ ionizing radiation. Ultrasound imaging can be used as a substitute for, or a complement to, various types of radiography (cone beam computed tomography or CBCT, panoramic x-ray, or intraoral x-ray imaging), magnetic resonance imaging (MRI), or nuclear medicine.

In order to observe structures, the ultrasound imaging may use high frequency soundwaves between 1 to 100 MHz. High frequency soundwaves may preferably be between 20 to 50 MHz for periodontal pocket investigation.

An ultrasound imaging apparatus generally comprises one or several transducers that act as ultrasound beam emitters and/or ultrasound beam receivers to receive echoes from the emitted signals. In addition, the ultrasound imaging apparatus may comprise various processing and display components used for generating and presenting images from acquired signals. An ultrasound beam emitter generates an ultrasound signal from an electrical signal and conversely, an ultrasound receiver generates electrical pulses from a mechanical ultrasound signal.

Objects in the path of emitted ultrasound signals return a portion of the ultrasound energy back to the transducer which generates electrical signals indicative of the detected structures. The electrical signals generated from the received ultrasound signal can be delayed for selected times specific to each transducer, so that ultrasonic energy scattered from selected regions adds coherently, while ultrasonic energy from other regions has no perceptible impact. Further, the emission of ultrasound signals can be delayed in order to enable adaptive focusing. The electronic adaptive focusing makes it possible to increase the resolution depending on the depth of the imaged organ.

Array processing techniques used for generating and processing received signals in this way are termed "beamforming".

Particular challenges with intraoral ultrasound imaging relate to the design of a probe that can be used for imaging a full set of intraoral structures, i.e. during periodontal examination, for positioning an ultrasound fan beam along the vertical axis of each tooth of a mouth on both buccal and lingual faces, without the need for extensive modification, reconfiguration, or changing of probe tips or other components. Indeed, to be efficient, the ultrasound probe window must be facing the regions to be imaged. The acoustic paths between the transducer and the regions to be imaged are ensured via coupling materials such as water-based gel with minimal attenuation.

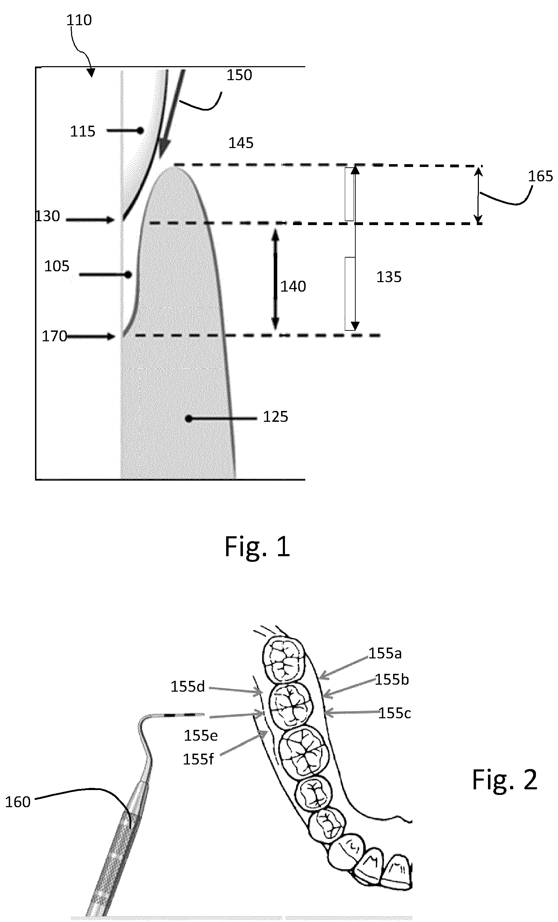

Periodontal check-ups or screening exams consist in measuring existing periodontal pockets to track periodontal diseases. **Figure** 1 illustrates an example of a dental structure 100 comprising a periodontal pocket 105. The teeth 110 are calcified structure having a crown surface covered with enamel 115 and roots attached to the bones of the superior or inferior jaws. Each tooth 110 is surrounded and supported by the periodontium, maintaining them in the maxillary and mandibular bones.

The periodontium comprises gingiva (gum) 125, surrounding the teeth 110 and providing a seal around them. The periodontium also comprises the periodontal ligament (not illustrated), the cementum (calcified substance covering the root of a tooth, not illustrated) and an alveolar bone proper (portion of bone on the jaws, maxilla and mandible, containing tooth sockets). The cementoenamel junction (CEJ) 130 is the anatomical border between the enamel 115 covering the crown of the tooth and the cementum covering the roots of the tooth 110. The CEJ 130 is also usually the area where the gingiva 125 attached to a healthy tooth by the gingival fibres.

A potential abnormality of the periodontium is the development of a periodontal pocket 105, meaning a space between the gingiva 125 and the tooth 110. The pocket 105 is characterized by a pocket base 170 and a cementoenamel junction (CEJ) 130, as illustrated in Figure 1. The pocket depth is the distance 140 between the pocket base 170 and the cementoenamel junction (CEJ) 130. When the top of gum 145 is vertically above the position of the CEJ 130, the distance 140 is not detectable by the standard periodontal probing and only the distance 135 is measurable and therefore considered as the pocket depth. At the base 170 of the periodontal pocket 105, the gingiva 125 is attached to the tooth 110 and forms the bottom of the periodontal pocket.

In order to measure the depth 135, 140 of a periodontal pocket 105, a periodontal probe may be used and inserted (as indicated by arrow 150), at least partly, inside the periodontal pocket 105, along the tooth 110. Ideally, it may be relevant to detect the CEJ 130 under the gum (fixed reference), as the distance 140 (attachment level) is the most relevant to quantify the pocket depth evolution. As illustrated in **Figure 2****,** in order to correctly assess the periodontal pocket depth 135 of a given tooth 110, the periodontal pocket 105 of one tooth 110 should be measured at several locations, for example six locations, on the periphery of the assessed tooth. In other words, using current probing systems (such as manual probe 160 as illustrated) in order to correctly examine the periodontal pockets 105 for a given tooth 110, different positions 155a, 155b, 155c, 155d, 155e, 155f (e.g. six) need to be checked for a single tooth 110.

Periodontal pockets 105 need to be regularly performed in order to check any infection of the gingiva 125. Further periodontal assessments may be accompanied with the measurement of the gingival regression 165 between the CEJ 130 and the top of the gum 145 (illustrated in Figure 1).

For a young adult with no periodontal predispositions, either the dentist chooses to perform a full periodontal examination, which is time-consuming and often involves at least two operators, or a partial periodontal examination with the risk of missing periodontal issues near the unexamined teeth.

Other abnormalities can develop in the periodontium or the gingiva area of the patients, such as tumours and/or abscesses. Currently, the dentist may detect such abnormalities either by observing the periodontium to the naked eye, or by biopsy. However, such methods are time consuming, and do not allows an almost immediate detection of abnormalities. The invention is directed to overcoming these drawbacks. The technical solution provided simplifies and speeds up periodontal screening examinations or regular periodontal or gingiva check-ups.

US 2017/0119505 discloses an apparatus for three-dimensionally measuring a row of teeth, that includes a measuring head comprising measuring members defining a variable measurement space. The measuring members can be moved in relation to each other to follow the shape of the teeth to be measured.

US 5,755 571 disclose an ultrasonic probe for detecting an abnormality of a periodontal pocket. The probe, using an ultrasonic transducer at its tip, is connected to a water source so that the periodontal pocket to be measured is filled with pressurized water.

### SUMMARY OF THE INVENTION

The present invention has been devised to address one or more of the foregoing concerns.
In this context, there is provided a method of detecting an abnormality along a portion of a dental arch using scanning material comprising an ultrasonic periodontal probe configured for emitting ultrasound signals within at least one emitting cone and for receiving corresponding echoed ultrasound signals, the method comprising moving the probe along a portion of the dental arch, wherein while moving the probe along the portion of the dental arch, following steps are performed by the probe:
emitting an ultrasound signal within the emitting cone;
measuring echoed ultrasound signals;
detecting at least one anatomical structure to be investigated;

the probe comprising a microcontroller configured for
   measuring at least one predetermined feature of the at least one detected anatomical structure to obtain a value of the measurement of the at least one predetermined feature characterizing the detected anatomical structure;
   detecting an abnormality as a function of a threshold and the value of the measurement of the predetermined feature of the at least one detected anatomical structure,
the probe further comprising at least an indicator configured to provide an item of information regarding an identified abnormality, the method further comprising energizing the indicator to indicate a detected abnormality.

Such a method enables the detection of abnormalities of the periodontium and/or the gingiva, using an ultrasonic periodontal probe during a fast scan of the patient gingiva or periodontium. Thus, the method helps to simplifies and speed-up periodontal examination, and also enables to detect abnormalities such as tumors. Therefore, during the same examination it may be possible to detect abnormalities of different natures.

According to some embodiments, detecting at least one anatomical structure to be investigated may comprise:
building images continuously based on the measured echoed signals;
analyzing the built images in order to detect at least one anatomical structure.

According to some embodiments, the physical quantity relating to the at least one detected anatomical structure may be one of a dimension, an echogenicity indicator, a texture inhomogeneity, a texture complexity.

According to some embodiments, analyzing the built images may comprise segmenting the built images for detecting at least one anatomical structure in the built images.

According to some embodiments, the at least one anatomical structure may comprise alone or in combination a tooth and/or a gum and/or a periodontal pocket and/or a cortical bone.

According to some embodiments, the physical quantity relating to a detected periodontal pocket may be a dimension such as depth.

According to some embodiments, each measurement may be notified to the operator while moving the probe along the portion of the dental arch.

According to some embodiments, a maximal measurement value and/or a minimal measurement value may be notified to the operator while moving the probe along the portion of the dental arch.

According to some embodiments, emitting an ultrasound signal may comprise recording an angulation of the probe and/or recording the position of the probe.

According to some embodiments, the ultrasonic periodontal probe may be configured for emitting ultrasound signals within at least one of several emitting cones extending in different directions.

According to some embodiments, emitting an ultrasound signal may comprise selecting at least one of the several emitting cones and emitting an ultrasound signal within the at least one selected emitting cone.

According to some embodiments, the indicator may comprise one or more light emitting devices and/or LCD screen and/or vibration means and/or sound effects.

### BRIEF DESCRIPTION OF THE DRAWINGS

Other features and advantages of the disclosure will become apparent from the following description of non-limiting exemplary embodiments, with reference to the appended drawings, in which:
**Figure** 1 is an example of a dental structure comprising a periodontal pocket;
**Figure** 2 is an example of locations for assessing a periodontal pocket according to prior art;
**Figures 3a to 3c** are examples of use of an ultrasonic imaging probe according to some embodiments of the invention;
**Figure** 4 is a schematic diagram illustrating an example of an electronic system of an ultrasonic imaging probe;
**Figures 5a** **and** **5b** are examples of the movement of a probe in order to perform a screening of the periodontium; and
**Figures 6** **and** 7 are examples of a protocol for screening periodontal pockets; and
**Figure** 8 is a diagram of an example of the method of scanning material **in order to** detect an abnormality along a portion of a dental arch according to some embodiments of the disclosure.

### DETAILED DESCRIPTION OF EMBODIMENTS OF THE DISCLOSURE

Some embodiments of the disclosure aim at providing a method using an ultrasonic probe for screening a periodontium and/or gingiva, for example for the development of periodontal pockets and/or tumoral masses follow-ups. The method according to the disclosure aims at performing a quick scan of the region of interest, computing and registering in real time a predetermined feature of an abnormality of a detected anatomical structure. The scan may be performed on an arch or a quadrant. For example, in the case of the periodontal pocket investigation in the periodontium, thanks to the scan, it may be possible to obtain the maximum value of the depth of a periodontal pocket in the assessed area. In that example, the scan is easily performed by an operator given that the probe movement is along the dental arch curve to the level of the periodontal area, and not along the gum line.

The following is a detailed description of particular embodiments of the disclosure, reference being made to the drawings in which the same reference numerals identify the same elements of structure in each of the Figures.

In the drawings and text that follow, like components are designated with like reference numerals, and similar descriptions concerning components and an arrangement or interaction of components already described are omitted. Where they are used, the terms "first", "second", and so on, do not necessarily denote any ordinal or priority relation, but may simply be used to more clearly distinguish one element from another, unless specified otherwise.

When referring to the shape of an apparatus, the term "rigid" should be understood as the substantially non-deformable nature of the apparatus, in normal use, which means that the relative position of main elements of the apparatus is substantially constant (i.e. there is no significant shape deformation during use). For example, the relative position of a grip portion, of a support member, and of an ultrasonic device of a rigid ultrasonic imaging probe is substantially constant when the imaging probe is used for imaging intraoral structures. This does not prevent the apparatus from being deformable during a configuration step, for example in a case in which two elements of the apparatus are fastened with a lockable hinge. In addition, this does not prevent the apparatus from comprising slightly deformable parts. For example, the grip portion of a rigid ultrasonic imaging probe may comprise a deformable handgrip such as a handgrip comprising elastic foam.

In the context of the present disclosure, the terms "viewer", "operator", and "user" are considered to be equivalent and refer to the viewing practitioner, technician, or other person who acquires, views, and manipulates an ultrasound image, such as a dental image, on a display monitor. An "operator instruction," "user instruction," or "viewer instruction" is obtained from explicit commands entered by the viewer, such as by clicking a button on the ultrasound probe or system hardware or by using a computer mouse or by using a touch screen or a keyboard entry.

In the context of the present disclosure, the phrase "in signal communication" indicates that two or more devices and/or components are capable of communicating with each other via signals that travel over some type of signal path. Signal communication may be wired or wireless. The signals may be communication, power, data, or energy signals. The signal paths may include physical, electrical, magnetic, electromagnetic, optical, wired, and/or wireless connections between the first device and/or component and second device and/or component. The signal paths may also include additional devices and/or components between the first device and/or component and second device and/or component.

The term "subject" refers to the tooth or other portion of a patient that is being imaged and, in optical terms, can be considered equivalent to the "object" of the corresponding imaging system.

### Periodontal probe

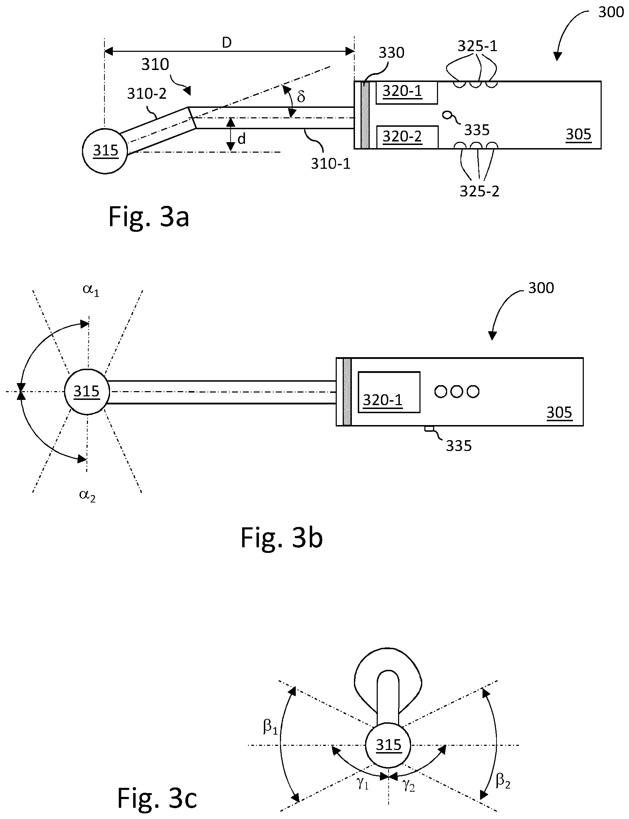

**Figures 3a, 3b** and **3c** are schematic representations of a rigid ultrasonic imaging probe that may be used in conjunction with particular embodiments of the method of the disclosure.

Figure 3a is a side view of the rigid ultrasonic imaging probe in a measurement configuration while Figures 3b and 3c represent a top view and a front view, respectively, of the same rigid ultrasonic imaging probe in the initial configuration. For the sake of the explanation, the initial configuration is distinguished from other measurement configurations, but it is not excluded that measurements are performed with the probe in the initial position.

The probe provides the ultrasound pulse signal emission and/or mechanical components for generating ultrasound beams in an emitting cone. The ultrasound beams may be cone-shaped beams or a fan-shaped beams contained in the emitting cone. The **cone-shaped** ultrasound beams may correspond to the emitting cone or be smaller than the emitting cone. Fan-shaped beams are planar.

The probe also provides acquisition logic for beamforming functions. The probe or a remote computer (not represented) obtains acquired signal data corresponding to received pulse echoes, processed or not, and renders images of the examined objects on a display that may be the LCD display of the probe or the remote computer display. The image content can also be stored for subsequent use or transmitted to another system or to a data storage apparatus.

As illustrated, the ultrasonic imaging probe 300 comprises a grip portion 305, a support member 310, and an ultrasonic device 315 (also referred to as ultrasonic sensor). According to the illustrated example, support member 310 comprises two main parts denoted 310-1 and 310-2, the longitudinal axis of part 310-2 being different from the longitudinal axis of the grip portion 305. It is to be noted that according to other examples, part 310-2 is fastened directly to grip portion 305 (i.e. without using part 310-1). Therefore, according to the illustrated example, ultrasonic device 315 is rigidly fastened to grip portion 305 in such a way that it is offset from the grip portion with regard to its longitudinal axis. As apparent from Figure 3a, ultrasonic device 315 is located at a distance d from the longitudinal axis (a) of grip portion 305. Distance d may be chosen between 0 and 5 centimeters. For the sake of illustration, it may be equal to 2 centimeters. Distance D between ultrasonic device 315 and grip portion 305 may be chosen between 4 and 25 centimeters. For the sake of illustration, it can be equal to 10 centimeters.

According to particular embodiments, support member 310 may comprise two tubular members forming an angle δ between their longitudinal axes, may comprise one or more tubular members and a portion of an annular member, or may comprise any composition of members making it possible to axially offset the ultrasonic device from the grip portion.

As illustrated, grip portion 305 may comprise elements enabling a user to interface with functions of ultrasonic imaging probe 300 and/or with a computer system processing signals acquired by ultrasonic imaging probe 300. Such elements may comprise displays generically referenced 320 (e.g. standard displays or touch screens) and buttons generically referenced 325. According to particular embodiments, all these elements or a subset of these elements may be duplicated (320-1 / 320-2 and 325-1 / 325-2) so that a user may interact similarly with ultrasonic imaging probe 300 and/or with a computer system processing signals acquired by ultrasonic imaging probe 300 whatever the position of the ultrasonic imaging probe 300 between a first and a second position (the second position being located on the opposite side of a horizontal plane relative to the first position).

In addition, grip portion 305 may comprise a set of LEDs (acronym of light emitting devices), for example the ring of LEDs 330. As described hereafter, these LEDs may be indicative of a selected emitting cone.

According to some embodiments, the LEDs or the ring of LEDs is arranged on the ultrasonic device or on the support member, close to the ultrasonic sensor.

As illustrated in Figure 3b, ultrasonic device 315 may be configured to make measurements according to two opposite directions, with reference to a vertical plane that is perpendicular to a horizontal plane comprising the longitudinal axis (a) of grip portion 305. Ultrasound fan beams may belong to emitting cones having angular cones α₁ and α₂ and angular cones β₁ and β₂, as illustrated in Figure 3b and Figure 3c, that may be in a range of 20° to 160°. Hereinafter, the expression "emitting cone" relates to the two angular cones constituting the emitting cones as described hereinbefore.

The transducers of the ultrasonic imaging probe 300 may be of the A-mode, B-mode (or 2D mode), C-mode, M-mode, Doppler mode, Color Doppler mode, Continuous Doppler mode, Pulsed wave Doppler mode, Duplex mode, Pulse inversion mode, or Harmonic mode type, each of them being well-known to the one skilled in the art.

According to some embodiments, each emitting cone is associated to a given transducer.

According to particular embodiments, the ultrasonic imaging probe 300 comprises a single transducer with a moving element (e.g. a moving transducer and/or a moving reflector), two or more transducers, or two or more arrays of transducers, arranged around an axis of the ultrasonic device that is parallel to the longitudinal axis of the grip portion, at least on each side of the ultrasonic device (when considering a measurement position of the probe 300). Thus, each position of the moving element (the transducer or the reflector) is associated to an emitting cone. In general, the moving element is rotatably mounted within a cavity of the ultrasonic device 315. The moving element is characterized by its acquisition scan direction that is the rotating direction of the moving element. The acquisition scan direction defines the orientation of the obtained images.

The transducers can act like an ultrasound beam emitter but also as an ultrasound beam receiver, configured to measure echoed ultrasound. Throughout the rest of the document, the transducers are both ultrasound beam emitters and receivers. In another embodiment, it may be considered that some transducers only act as ultrasound beam emitter or ultrasound beam receiver.

When acting as an emitter, the transducer is associated with an emitting cone that defines the volume, in which the associated transducer is able to emit an ultrasound signal.

When acting as a receiver, the transducer is passive and is configured to act as a sensor and to detect and measure any received echoed ultrasound signals. In other words, the transducer detects and measures the incidents waves.

According to some embodiments, each transducer may comprise several emitting elements and/or several receiving elements. Having several receiving elements, helps improving ultrasound beam reception while reducing the effect of noise on the received ultrasound signals.

It is noted that although the directions of the two emitting cones (i.e. the cones in which ultrasound fan beams may be emitted) represented in Figures 3b and 3c are perpendicular to the longitudinal axis of grip portion 305 and to a vertical plane, these directions may be different. These directions may form angles θ₁ and θ₂ with regard to the longitudinal axis of grip portion 305 and angles γ₁ and γ₂ with regard to a vertical axis perpendicular to the longitudinal axis of grip portion 305, respectively, varying from about 20° to 160°, provided that these directions are opposite to each other, respectively, with regard to a plane comprising the longitudinal axis of grip portion 305.

According to some embodiments, the probe may be configured for emitting ultrasound signals within one emitting cone. Consequently, the ultrasound signal is emitted along one direction, which is comprised into the one emitting cone of the ultrasonic device 315.

### Electronic elements of the ultrasonic imaging probe

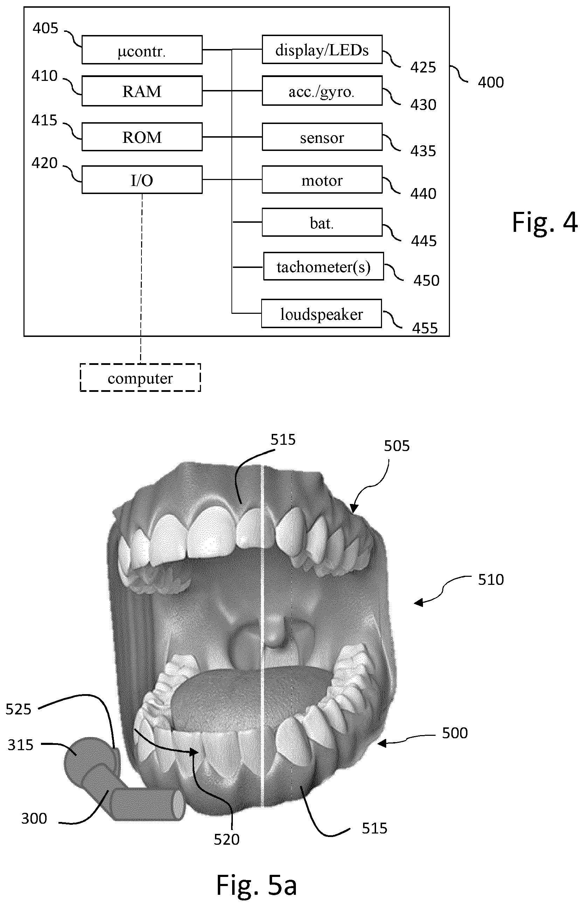

**Figure** 4 is a schematic diagram illustrating an example of an electronic system of an ultrasonic imaging probe 300.

As illustrated, the electronic system 400 comprises buses and/or electrical connections connecting:
- a microcontroller 405;
- a random access memory 410, denoted RAM, for storing the executable code for operating the ultrasonic imaging probe 300 as well as the registers adapted to record variables and parameters;
- a read-only memory 415, denoted ROM, for storing computer programs for operating the ultrasonic imaging probe 300 and/or configuration parameters;
- an input/output interface 420 to set the ultrasonic imaging probe 300 in signal communication with a remote computer, for interfacing the ultrasonic imaging probe 300 with the remote computer, for example to transmit measured signals and/or receiving commands for controlling some of the operations of the ultrasonic imaging probe 300, the input/output interface being a wired or wireless interface, for example a wireless interface complying with the WiFi and/or Bluetooth standards (WiFi and Bluetooth are trademarks);
- a display and/or LEDs 425 for giving indications to the user, for example the state of the ultrasonic imaging probe 300, a step or a next step to do, and/or the direction according to which measurements are made or will be made, or the measurement value, or whether one measurement value is greater than the threshold;
- a motion sensor, a position sensor, and/or an orientation sensor 430 comprising, for example, an accelerometer and a gyroscope, making it possible to determine the position, the orientation, the speed, and/or the acceleration of the ultrasonic imaging probe 300, which may be used, for example, to identify commands by gesture type (e.g. a particular gesture may be used to select the directions according to which measurements are to be made);
- an ultrasonic device 435 comprising one or more transducers or arrays of transducers as described above;
- a motor 440 for moving parts of the ultrasonic sensor, for example a transducer, an array of transducers, or a reflecting element;
- a battery 445, rechargeable or not, for providing electrical power to the components of the electronic system;
- one or several tachometers 450, for indicating for example the current value and/or the maximum measurement and/or the minimum measurement, such as a measured depth of a periodontal pocket and/or the measured dimension and/or the texture complexity and/or the texture inhomogeneity of the tumoral mass; and
- a loudspeaker 455, for indicating the detection of an abnormality along the screened portion.

It is to be noted that the ultrasound imaging device may comprise other electronic elements such that some of the electronic components mentioned above may not be required. For example, a motor is not required if the ultrasonic imaging probe 300 comprises several fixed transducers or arrays of transducers. Likewise, internal electrical power may not be required if the ultrasonic imaging probe 300 is connected to a remote computer via wires.

According to some embodiments, the executable code may be stored either in read-only memory 415 or on a removable digital medium such as for example a micro memory card. According to a variant, the executable code of the programs can be received from the remote computer via the input/output interface, in order to be stored in one of the storage means of the communication device 400.

Microcontroller 405 is adapted to control and direct the execution of the instructions or portions of software code of the program or programs for operating the ultrasonic imaging probe 300 according to particular embodiments of the invention, the instructions being stored in one of the aforementioned storage means. After powering on, microcontroller 405 is capable of executing instructions from main RAM memory 410 relating to a software application after those instructions have been loaded from ROM 415 or from a remote computer for example.

Microcontroller 405, RAM 410, and/or ROM 415 may be implemented in hardware by a machine or a dedicated component, such as an FPGA (Field-Programmable Gate Array) or an ASIC (Application-Specific Integrated Circuit).

Upon reception, the acquired ultrasound signals may be processed, for example filtered, by electronic system 400, for example by microcontroller 405, before being transmitted to the remote computer through input/output interface 420. According to particular embodiments, the acquired ultrasound signals may be processed to generate images that are transmitted to the remote computer. Alternatively, the acquired ultrasound signals may be transmitted directly to the remote computer as raw data. The processing power and capabilities of electronic system 400 may depend on the processing to be applied to the acquired ultrasound signals.

The motion sensor and/or orientation sensor may be used to detect predetermined commands. For example, a movement of the ultrasonic imaging probe 300 to the right may indicate that measurements are to be made on the right side of the ultrasonic imaging probe 300. Likewise, a movement of the ultrasonic imaging probe 300 to the left may indicate that measurements are to be made on the left side of the ultrasonic imaging probe 300.

The motion sensor and/or orientation sensor may also be used to determine the position of the ultrasonic imaging probe 300 with regard to a default position. The determined position of the ultrasonic imaging probe 300 may be used to appropriately orient images generated from received ultrasound signals.

As described above, an indication may be displayed on a display or as a subset of one or more LEDs to provide an indication regarding the direction according to which measurements are made. According to some embodiments, piezoelectric elements surrounding the reception area are used to generate energy for energizing local indicators (for example small LEDs) in the vicinity of the probe tip 315.

To that end, piezoelectric elements, such as those used in transducers, having the capability to both generate a mechanical wave due to electrical excitation and, conversely, to generate an electrical pulse once placed under mechanical stress, are used. When using the ultrasonic imaging probe 300, a transducer generates a number of ultrasound waves that are reflected and deviated by the observed tissues. During signal acquisition, for generating images, incident waves not only impact the transducer but also affect areas outside the transducer surface, including portions of the piezoelectric material not used for capturing signals, called piezoelectric wells for the sake of clarity. Therefore, it is possible to use part of the excess energy that is otherwise lost, in the vicinity of the emitters and receivers actually used to make measurements, by considering a number of piezoelectric sources for energy harvesting. These piezoelectric wells can capture excess energy that is not captured as part of the reflected acoustic signal and generate sufficient current to energize small LEDs and associated capacitive elements. As described above, the generated illumination from this energy harvesting can provide information to the user, to visually highlight the position or angular orientation of the emitted ultrasound beam. The generated illumination may even be strong enough to illuminate the scanned area.

According to some embodiments, the probe may be autonomous. In other words, the probe may be usable without any other systems, such as a remote computer. In this case, the probe may be configured to emit ultrasound signals, to acquire echoed ultrasound signals, to build an ultrasound image, to perform measurements and then to display notifications on the LCD display of the ultrasound probe. Consequently, the probe may comprise a microcontroller associated to memory (such as RAM and ROM) configured to process the acquired echoed ultrasound signals. Thus the microcontroller of the probe is configured to execute the instructions of the software application recorded the embedded memory of the probe. Such a probe is then autonomous for the reconstruction of the ultrasound images, measurement of the echoed ultrasounds and notification of the measurements and/or the reconstruction to the operator.

### Initialization and positioning of the probe for screening a region of interest

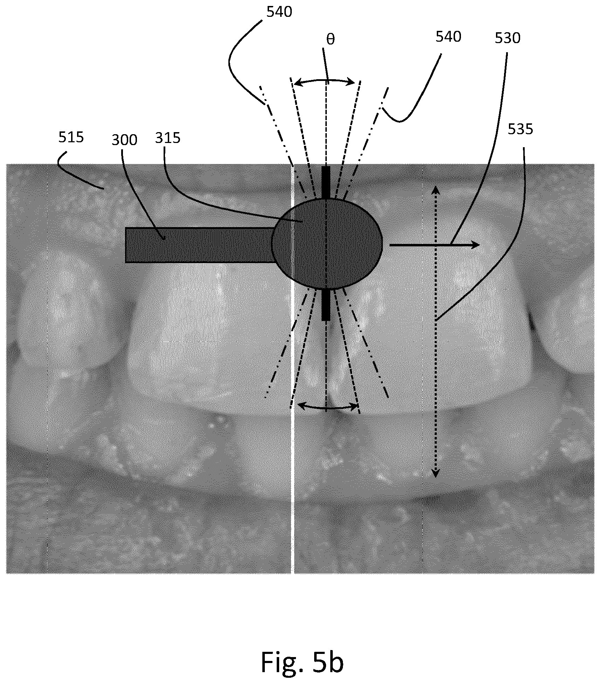

**Figures 5a** **and** **5b** illustrate examples of uses of an ultrasonic imaging probe according to particular embodiments of the disclosure, in order to screen a portion of the dental arch comprising soft and hard tissues.

Figure 5a illustrate the movement of the probe 300, when it is used to screen a region of interest in a patient mouth. The operator handles the grip portion 305 of the probe 300 and arranges the ultrasonic device 315 of the probe 300 in order to observe the region of interest.

An example of an area of interest for detecting abnormalities is the gingiva area 515, indicated in Figure 5a. The gingiva area 515 can be defined as the visible part covering the periodontia. This area also corresponds to a sector of the mouth in which the periodontal pockets are likely to develop. As explained hereinbefore in reference to Figure 1, a periodontal pocket 105 may appear between the tooth 110 and the gum 125 in the circumferential area of the tooth 110 where the gum 125 usually surrounds the tooth 110 in collar-like fashion. The gingiva area 515 is a sector extending along both dental arch 500, 505. The dental arches 500, 505 are the two arches that contain teeth 110, one of each jaw, that constitute together the dentition 510. The upper dental arch 505 of the maxilla (or upper jaw) and the lower dental arch 500 of the mandible (or lower jaw) respectively comprises periodontia which maintain the teeth on the maxilla and the mandible.

The same gingiva area also corresponds to a sector of the mouth in which tumoral masses are likely to develop. Indeed, the tumoral masses may appear in the gingiva epitheliums. A tumoral mass may be detected using the ultrasound probe when moved along this area. Indeed, the detection of a change in the echogenicity response of the raw echoed ultrasound signals or a specific mass visible in the reconstructed image may allow a tumoral mass to be detected.

As visible in Figure 5a, each dental arch 500, 505 is an arc, i.e. having a curve
In order to observe a portion of the gingiva area 515, the ultrasonic device 315 is arranged such that the cone-shaped or fan-shaped beam emitted within the emitting cone is reflected on the portion of the area of interest to be observed.

To do so, the ultrasonic device 315 may be arranged such that the portion of the area of interest is contained in the emitting cones and/or is intersected by a plane of the emitting cones containing the fan-beam, further explained in relation with Figure 5b.

Moreover, as the gingiva area 515 extends along the dental arch 500, 505, in order to have a comprehensive investigation of the gingiva area 515, the operator may put in motion the ultrasonic device 315 in order to obtain several ultrasonic signals and/or reconstructed images along the dental arches 500, 505.

In other words, when holding the probe 300, the operator moves the probe 300 along a portion of the gingiva area 515 to be observed.

An example of the movement of the probe for obtaining images of the lower gingiva area of the mandibula is illustrated in Figure 5a, by the arrow 520. As visible in Figure 5a, the ultrasonic device 315 is arranged such that a transducer 525 faces the gingiva area 515 and is put in motion along the dental arch following arrow 520.

Figure 5b illustrates the positioning of the ultrasonic device 315 of the probe 300 in order to measure anatomical structures of the gingiva area 515, along the dental arches 500, 505.

During the acquisition process, in order to ensure that the measurements of anatomical structures are correctly performed, the probe 300, particularly the emitting cone of the probe 300, is positioned in relation with the dental arch 500, 505 where the gingiva area 515 to be observed is.

To do so, as visible of Figure 5b, the angular position of the probe 300, according to the tooth axis 535, could be monitored. Thus, to obtain correct measurements of the given periodontal pocket 105, the operator holds the probe 300 such that the ultrasound beams to observe the gingiva area 515 may be as much as possible orthogonal to a tangent vector 530 of the dental arch curve and following the teeth axis 535. In order to ensure usable measurements, a threshold, corresponding to the maximum deviation angulation 540 of the ultrasonic device 315 may be specified.

While screening for the detection of tumoral mass, the angulation monitoring is not required. Indeed, as tumoral masses do not have a preferred direction of investigation, the benefit of the monitoring of the probe positioning seems limited.

According to some embodiments, the maximum deviation angulation threshold may be provided by a software application hosted on the remote computer connected to the probe 300 (through an input/output interface)/ or hosted on the probe itself.

The maximum deviation angulation threshold may be a deviation angulation in relation to an initial position of the probe. For example, the software application comprises an initialization procedure, consisting in instructing the operator to position at least one emitting cone of the probe in the right position, in front of the most accessible tooth of the patient (for example one of the incisors). The angulation of the initial position of the probe is then registered.

Next, during the acquisition process, the deviation angulation of the probe is monitored by the software application, such that as soon as the angulation of the probe 300 is greater than a maximum deviation value, the software application notifies the operator. The maximum deviation value could be set to less than or equal to 15°preferably less than or equal to 10°, more preferably less than or equal to 5° to guarantee accurate measurement along the periodontal pocket axis. According to some embodiments, the live deviation angulation of the probe, during the patient examination, may be recorded, transferred or displayed on the LCD screen of the probe or on the screen of the computer (of the remote workstation).

According to some embodiments, the software application may comprise features that enable the obtained images of the patient's mouth associated with an angulation deviation greater than the maximum deviation value to be automatically or semi-automatically discarded.

According to some embodiments, the angulation of the probe 300 is monitored thanks to the sensor 430 mentioned with reference to Figure 4.

### Screening of a region of interest of a dental arch

As detailed herein, a probe 300 adapted for screening a dental arch advantageously comprises an ultrasonic periodontal probe 300 configured for emitting ultrasound signals within an emitting cone and for receiving corresponding echoed ultrasound signals, as illustrated in Figures 3a, 3b, 3c, 4, 5a and 5b.

When using a probe 300 having two or more emitting cones extending in different directions, in order to reduce power consumption and to improve the quality of the results by avoiding parasitic signals, it is advantageous to select the emitting cone(s) to be used. The selection may be manual or automatic.

However, in order to reduce the time of a periodontal examination or an oncological examination it is advantageous to propose the detection of abnormalities of the periodontium or the gingiva epitheliums, while moving the probe continuously along the portion of the dental arch, in the gingiva area.

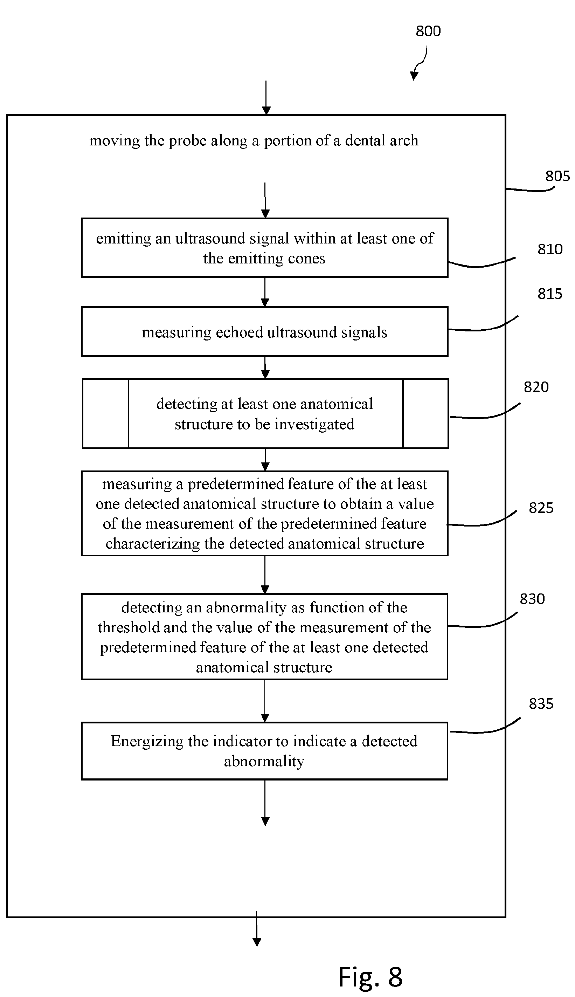

**Figure** 8 is a diagram illustrating an example of steps of a general method for scanning material in order to detect an abnormality along a portion of a dental arch according to some embodiments of the disclosure.

As illustrated, the general method 800 is performed while the probe 300 moves along a portion of the dental arch 805 comprising a plurality of teeth such as the one illustrated in Figure 5a.

As explained in reference to Figure 5b, according to some embodiments, the method may be performed after the user executes the initialization procedure as described above to register the initial position of the probe.

The method 800 comprises a step of emitting an ultrasound signal within at least one of the emitting cones 810 facing the tissue to be observed.

According to some embodiments, the step of emitting is performed as a part of an acquisition process. The acquisition process may be free, semi-automatic or automatic as detailed hereinafter.

When using the probe illustrated in Figure 3a that has two emitting cones, the step of emitting may comprise a step of selecting at least one of two emitting cones and then emitting an ultrasound signal within the selected emitting cone.

The same applies when using a probe having several emitting cones extending in different directions.

In order to avoid an interference between ultrasound signals emitted within two different emitting cones, the probe may be configured to select only one emitting cone at a time in both emission and reception modes. An acquisition cycle (i.e. the emission of ultrasound signal and the reception of echoed ultrasound signal) must be completed before starting another acquisition cycle involving either the same or a different emitting cone. The probe may be configured in order to ensure that an acquisition cycle is completed before starting another.

Thus, while the probe moves from area to area, tooth by tooth, ultrasound signals are continuously emitted 810, and echoed ultrasound signals are continuously measured 815 by the transducers of the probe.

Steps 810 and 815 form together an acquisition cycle. During the acquisition process, several acquisition cycles may occur.

Next, the method comprises a step of detecting at least one anatomical structure to be investigated 820.

According to some embodiments, to do so, images of the scanned area (tooth after tooth) are built continuously based on the measured echoed signals, and then analyzed. The analysis allows the detection of abnormalities of the region of interest in the gingiva area, such as periodontal pockets of the periodontium or masses having an echogenicity different from the one of the gingiva's epitheliums.

According to some embodiments, the analysis of built images may comprise two consecutive steps. The first step is to filter the built images for identifying anatomical structures of the built images. This step aims at identifying the objects that are visible in the built images, meaning anatomical structures such as teeth, gum, gingiva's epithelium, cortical bone, and periodontal pockets.

According to some embodiments, depending on which anatomical structures the operator would like to observe, the step of filtering may be used to identify the meaningful images. For example, when screening for periodontal pockets, only the image in which periodontal pockets have been detected, are subsequently used in the method. Same applies to ultrasonic images not presenting tumoral tissues in case of oncological examination. Thus, images with no interest for the operator may be automatically discarded without any notification to the operator.

Next, the method comprises a step 825 of measuring a predetermined feature of the at least one detected anatomical structure to obtain a value of the measured predetermined feature characterizing the structure.

The predetermined features may be dependent on the detected anatomical structure. Indeed, whether the detected anatomical structure is a periodontal pocket, the predetermined features may be the depth or any other dimensions of the detected periodontal pocket features. Whether the detected anatomical structures are rather the gum and gingiva's epithelium, the predetermined features may be the echogenicity, the textures complexity and inhomogeneity. Indeed, tumoral masses, likely to appear in the epithelium of the gingiva, have a different echogenicity, and different textures complexity and inhomogeneity from a healthy epithelium of the gingiva.

In other words, the step 825 aims at the measurement of features of the anatomical structure previously detected (i.e. for example, measurement of a depth of an identified periodontal pocket or of a texture complexity of an epithelium of a gingiva). To do so, measurements are performed on the built image in which the structure to be characterized have been detected (in step 820).

Next, the method 800 comprises a step 830 of detecting an abnormality as function of the threshold and the value of the measurement of the predetermined feature of the at least one detected anatomical structure.

This threshold may be predetermined or set by the operator through an input/output interface of the software application hosted on the remote computer connected to the probe or directly through the device human interface displayed on the LCD of the probe. The threshold may be determined dynamically during the screening examination.

When multiple type of anatomical structures are investigated during the same screening examination, several thresholds may be defined and changed by the operator, each threshold being associated to a specific type of anatomical structure (maximum of periodontal pocket depth, maximum/minimum texture inhomogeneity of soft tissues).

Thus, the value of each measurement of the predetermined feature of the detected anatomical structure may be compared to a threshold. An indicator of the probe may be configured to provide an item of information regarding an identified abnormality. For example, when a portion of the gingiva having an echogenicity greater than a threshold is detected, the indicator may be actuated to notify the possible presence of a tumor. Same applies to a periodontal pocket having an abnormal depth.

Next, according to some embodiments, the method further comprises a step 835 of energizing the indicator to indicate abnormalities of the region of interest in case of detection. The indicator may be one of the light emitting devices 425 of the probe, the LCD screen 425, vibration means or a loudspeaker 455 as described in reference to Figure 4.

For example, the operator may be notified by the system using an alert message displayed on the computer display and/or LCD display of the probe, the vibration of the probe, the illumination of at least one of the LEDs (continuously or discontinuously, with color variations), or the diffusion of sound effects through the loudspeaker.

Once the steps of the general method 800 has been performed, the software application hosted in the remote computer or in the microcontroller embedded in the probe, may instruct to record the acquisition data. According to some embodiments, all the acquisition data or only acquisition data with interest may be recorded. For example, only data relating to a periodontal pocket having a depth exceeding the periodontal pocket threshold or the detection of a tumor mass having a texture inhomogeneity and/or complexity greater than the threshold may be recorded. According to some embodiments, both of the anatomical structure measurement and associated image(s) (used for measurements) may be archived in the computer, including optional notes of the operator and/or the system (such as the tooth number, the probe positioning, the probe angular orientation).

According to some embodiments, while moving the probe tooth by tooth, the angulation of the probe is monitored and continuously compared to the initial position, in order to ensure the measurements are correctly performed. Consequently, during the acquisition process, an angulation deviation of the probe may be monitored, and also recorded as optional notes of archived images.

Thanks to this method, while moving the probe along a portion of the dental arch, the probe collects images of the gingiva area continuously and without interruption. Consequently, in the case of periodontal check-up, the time to perform a periodontal assessment is significantly shortened.

### Manual screening of a region of interest of a dental arch

In the manual mode, the operator freely moves the probe along one or several portions of the dental arches that he or she chooses. In other words, during a gingiva area assessment, the operator may freely choose the areas to be assessed, in order to complete a periodontal pocket and/or a tumoral masses assessment. In this case, the operator freely investigates the gingiva areas in the patient's mouth.

First, the operator selects and actuates the transducer emitting ultrasound signals to observe the anatomical structures of the region of interest of the patient.

According to some embodiments, when using a probe having one emitting cone, the operator may actuate the transducer in order to make it emit ultrasound signals. Then, the method 800 may be performed, such that the probe continuously allows several images of the portions of the dental arch to be obtained.

In other words, when exploring portions of the gingiva areas of the dental arches of a patient, the operator may obtain several images of the portions, which are automatically analyzed and interpreted thanks to the steps 820, 825 and 830 of the method 800 (for example, for assessing the depths of detected periodontal pockets or for detecting the presence of tumoral masses).

According to some embodiments, the operator may turn the probe off when moving it between anatomical regions in the patient mouth that are far apart.

According to some embodiments, when moving the probe between anatomical regions that are far apart, the method may be continuously performed, and images wherein no anatomical structures of interest are detected during the step of filtering may be automatically or semi-automatically discarded.

According to some embodiments, when using a probe such as the one illustrated in Figure 3a, 3b and 3c, the operator may actuate a transducer to make it emit ultrasound signals in the associated emitting cone, possibly after selecting one emitting cone from several emitting cones of the probe. The operator may select the emitting cone according to the area of interest to be observed. Thus, when exploring the intraoral cavity of a patient, the operator may change the actuated transducer by another whose arrangement is better to observe the area of interest.

According to some embodiments, the actuation of the transducer(s) results from an actuation of a physical button arranged on the ultrasonic imaging probe 300. As described in relation with Figures 3a, 3b and 3c, each button 325-1, 325-2 can be configured to be linked to a transducer associated with an emitting cone.

In the case of the touch-sensitive screen, the user may select one transducer using a finger to indicate the transducer to be used.

According to some embodiments, the operator may actuate the transducer(s) by choosing an item displayed on either the LCD screen of the probe or a software graphic interface hosted on a computer connected to the ultrasonic imaging probe 300. As illustrated in Figure 4, the electronic system of the imaging probe 300 can be interfaced with a remote computer (comprised in a remote workstation) through an input/output interface. In this case, the computer hosts a program configured to operate the probe 300, and particularly to indicate in which direction the user wants to make measurements, as described hereinbefore for the touch screen.

According to some embodiments, the actuated transducer associated with the selected emitting cone may be indicated to the operator, for example with the help of LEDs on the probe 300 and/or on the acquisition interface of the remote computer.

Once one of the transducers has been actuated, the operator moves the probe along the dental arch, and the method 800 is performed in order to screen the region of interest of the dental arches of the patient. As explained hereinbefore, after detecting abnormalities, an indicator of probe may be energized to indicate identified abnormalities in the region of interest investigated. The indicator may be one of the light-emitting devices 425 of the probe, the LCD screen 425, vibration means or the loudspeaker 455 as described in reference to Figure 4.

For example, the operator may be notified by the system using an alert message displayed on the computer display and/or LCD display of the probe, the vibration of the probe, or illumination of one of the LEDs (continuously or discontinuously, or with color variations), or a sound effect diffused through the loudspeaker.

### Semi-automatic or automatic screening of a region of interest of a dental arch

In the semi-automatic and automatic screening of a region of interest (such as the gingiva area), the operator is guided by the software application through the acquisition process. The software application comprises acquisition sequence (scenarios), indicating the order in which the areas are scanned, meaning at which dental arch location the acquisition may begin and end (for example a given tooth for a periodontal assessment).

Several acquisition processes may be available to the user and may offer to fully screen the gingiva area of a dental arch, or a portion such as a hemi-dental arch or a dental arch quadrant. Of course, the software application may allow the customization and/or the creation of acquisition processes by the operator.

According to the anatomical structures to be assessed, several acquisition processes may be available. The software application may guide the user to scan the expected areas.

Thus, acquisition processes for periodontal examination and for oncological examination may be different.

According to some embodiments, once the user chooses an acquisition process on the software graphic interface, the software application may actuate one of the transducers of the probe to make it emit ultrasound signals within the associated emitting cone. The actuated transducer is then indicated on the software graphic interface to help the operator to correctly position the probe.

According to some embodiments, the actuated transducer associated with the selected emitting cone may be indicated to the operator, for example with the help of LEDs on the probe 300 and/or on the acquisition interface of the remote computer.

Thus, the operator holding the probe 300 is then guided by the software application through the acquisition process. The software application may provide protocols ensuring the scan of a patient's intraoral cavity in the most efficient way, by limiting switching from one emitting cone to another. While the operator moves the probe according to the chosen protocol, the method 800 is performed.

According to some embodiments, after the anatomical structure assessment in manual or automatic mode, for each predetermined feature, the maximal and/or minimal measured feature values of the screened areas is/are displayed, either on the computer screen or the LCD screen of the probe.

### Example of a protocol for the automatic screening of abnormal periodontal pockets

When the method 800 is performed in order to detect abnormal periodontal pockets, the predetermined feature may be for example the depth of the periodontal pocket, as explained in the description in relation Figure 1. Of course, other dimensions of the periodontal pockets may be used as predetermined features.

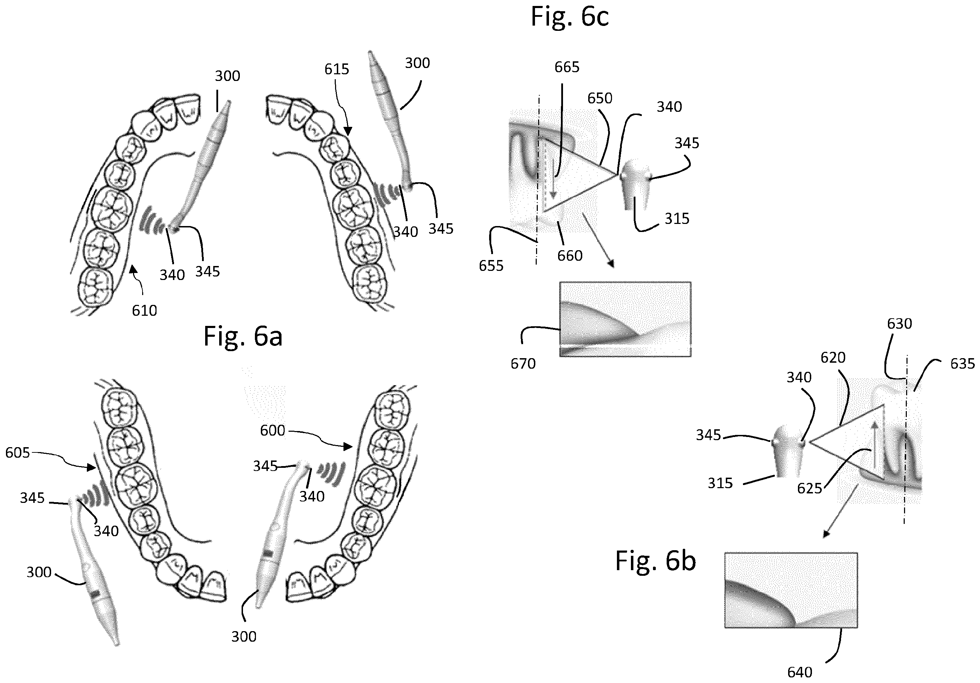

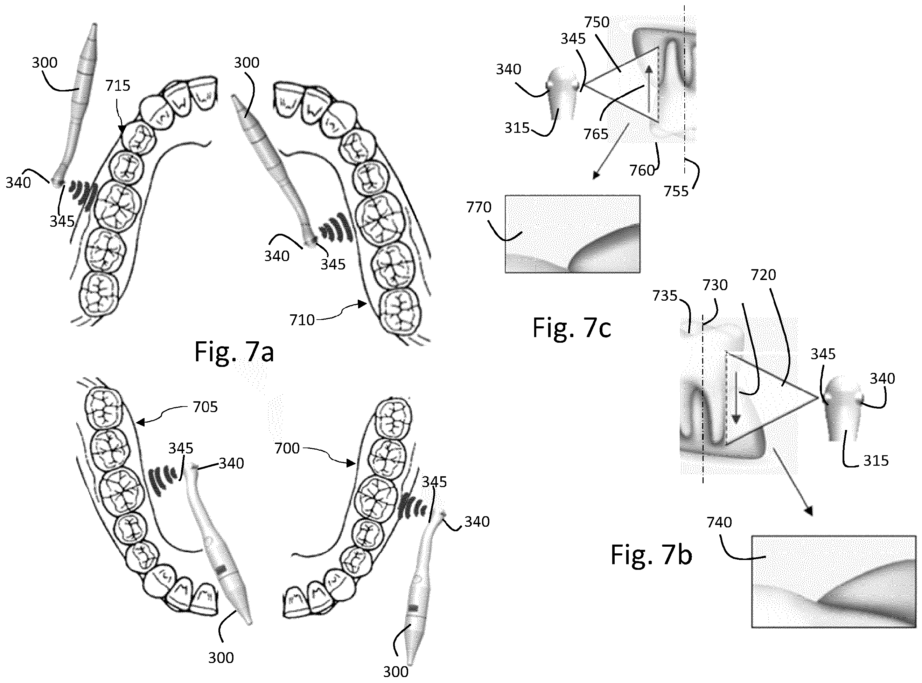

An example of a protocol optimizing the duration of a periodontal pocket assessment is illustrated in Figures 6a, 6b, 6c and 7a, 7b, 7c using a probe 300 configured for emitting in two emitting cones, extending in opposite directions such as the one described in reference to Figures 3a, 3b and 3c.

The probe therefore comprises an ultrasonic device 315 comprising two transducers 340, 345 that may be actuated by the software application configured to operate the probe 300 and to cause the transducers to emit ultrasound signals.

The illustrated protocol is carried in two phases, respectively illustrated in Figures 6a, 6b, 6c and Figures 7a, 7b, 7c, respectively corresponding to the actuation of transducers 340 and 345.

First, the transducer 340 is actuated by the software application, and the operator start moving the probe. The protocol indicates to the operator to first move the probe 300 along the gingiva area arranged on the left lingual mandibula 600, and then along the one arranged on the right vestibular mandibula 605. For each dental arch quadrant 600, 605, the operator may start from the bottom tooth of the quadrant and move the probe 300 tooth by tooth, to scan entirely these two areas 600, 605.

When moving the probe 300 along these two areas 600, 605, the method 800 is performed, such that several images of the areas are continuously obtained, and existing periodontal pockets or tumoral masses are measured. Figure 6b illustrates the positioning of the emitted ultrasound fan beam 620 in relation a given tooth 635 of either the left lingual mandibular 600 or the right vestibular 605 in order to obtain workable images used for measurements of biological structures.

In the context of a fast periodontal check-up, the probe 300, in this example, emits an ultrasound fan beam 620, extending in a plane parallel to the axis 630 of the tooth. Therefore, the ultrasound fan beam 620 is sensibly orthogonal to a tangent vector of the considered dental arch curve. The vector 625 represents the acquisition scan direction in case of fan beam ultrasonic signal generated by a moving element (transducer or deflector).

Obtained image 640 is an example of an image in which the periodontal area of the given tooth 635 may be observed. As can be seen, the obtained image 640 shows the area where the gum surrounds the tooth 635, i.e. the area where the periodontal pockets are likely to form.

Next, the operator starts moving the probe 300 following the protocol that indicates to the operator to move the probe 300 first along the gingiva area on the right lingual maxilla 610 and then along the one on the left vestibular maxilla 615. The movement of the probe 300 is the same as the one described in relation with the scan of the quadrant 600, 605 of the dental arches.

The transducer 340 emits an ultrasound fan beam 650, oriented similarly to the fan beam 620 in relation to the tooth axis 655, and is illustrated in Figure 6c in relation with a tooth 660 of either the right lingual maxilla 610 or the left vestibular maxilla 615. The ultrasound fan beam 650 is sensibly orthogonal to a tangent vector of the considered dental arch curve. The vector 665 represents the acquisition scan direction in case of fan beam ultrasonic signal generated by a moving element (transducer or deflector).

An example of the obtained image 670 shows the periodontal area of the scanned tooth 660.

The second phase of the protocol aims at measuring the remaining parts of the dental arches, with the second transducer 345.

First, the transducer 345 is actuated by the software application, and the operator start moving the probe 300. The protocol indicates to the operator to first move the probe 300 along the gingiva area on the left vestibular mandibula 700, and then along the one on the right lingual mandibula 705. Similarly, as described in reference to Figures 6a, 6b, 6c, the operator moves the probe 300 starting from the bottom tooth of each quadrant, tooth by tooth, while maintaining the probe in a correct position to obtain workable images. A correct position of the probe, for the periodontal pocket investigation, is illustrated in Figure 7b illustrating the ultrasound device 315 of the probe 300 emitting an ultrasound fan beam 720 arranged such that the ultrasound fan beam is parallel to the axis 730 of the tooth 735 and orthogonal to a tangent vector to the dental arch curve.

The protocol next indicates to the operator to move the probe 300 along the gingiva area on right vestibular maxilla 715, and then along the one on the left lingual maxilla 710.

The operator respectively moves the probe along these areas with the ultrasound signal provided by the transducer 345.

Examples of obtained images 740 and 770 show the periodontal area, more particularly the area where the gum surrounds the teeth 735, 760.

This illustrated protocol allows the limitation of the number of times the software application has to switch between the transducers 340, 345.

During the protocol of assessment, the software graphic interface may comprise indication regarding the movement of the probe that the operator should perform. Furthermore, the software application may notify the user when the probe positioning, especially the angulation of the probe, does not allow correct measurements of the biological structures that may be detected in the obtained images. The monitoring of the probe deviation may be performed during the anatomical structure assessments, as described hereinbefore in reference to Figure 5b.

Therefore, the scanning process is optimized and the amount of time to do a complete examination of a patient's intraoral cavity is reduced.

According to some embodiments, the protocol may specify to perform the measurements of the vestibular parts of the upper and lower dental arches, and then the lingual parts of the upper and lower dental arches.

According to some embodiments, the software application may allow additional protocols to be established by the operator.

As explained hereinbefore, after detecting abnormalities of the region of interest investigated, an indicator of probe may be energized to indicate identified anatomical structure abnormalities. The indicator may be one of the light emitting devices 425 of the probe, the LCD screen 425, vibration means and the loudspeaker 455 as described in reference to Figure 4.

For example, the operator may be notified by the system using an alert message displayed on the computer display and/or LCD display of the probe, the vibration of the probe, or illumination of at least one of the LEDs (continuously or discontinuously, or with color variations), or a specific sound effect diffused through the loudspeaker.

Of course, with the help of physical buttons and/or on the graphical interface of the software application operating the probe 300, it is possible for the operator to switch between the manual, semi-automatic and automatic modes.

### Screening of tumoral masses in a patient mouth

During the screening of tumoral masses, several predetermined features may be measured.

As mentioned hereinbefore, when the method is performed to detect tumoral masses during the screening examination, the predetermined features of the detected soft tissues, i.e. gum, epithelium of detected gingiva, are thus measured.

The predetermined features may be for example an echogenicity indicator, a texture inhomogeneity, a texture complexity.

An echogenicity of a tissue is the ability of the tissue to bounce an echo. Thus, when the amplitude of the ultrasound signal reflected by the tissues is high, these tissues have a high echogenicity indicator (hyperechoic). These tissues are then represented with lighter colors in the built images from echoed ultrasound signals. Conversely, the tissues are hypoechoic when the amplitude of the reflected ultrasound signal is low. For example, an unusual contrast variation in the built images may therefore indicate the presence of a tumor mass. In diseased state, the echogenicity of an organ can be altered and become more or less echogenic (hyperechoic or hypoechoic).

The complexity and/or the inhomogeneity of the texture of the tissues may also characterize the internal structure of the tissues constituting the tumor mass. For example, the complexity of a tumoral mass can be appreciated by the fractal dimension of the texture. These two features, individually or together, enables the texture of tissues constituting the tumor to be assessed. Further, these features permit the tumor evolution to be followed over time.

Altogether, the predetermined features, echogenicity, texture complexity and texture inhomogeneity, may help to characterize a tumor mass when screening the region of interest with the probe.

Consequently, it may be possible, according to some embodiments, to perform the measurements of these three predetermined features, and to compare the value of the measurements respectively to three threshold associated to each predetermined features.

Thus, a tumoral mass may be detected and characterized as a function of the values of measurements of these predetermined features and the thresholds.

### Screening of abnormal several types of anatomical structures (that may be either periodontal pockets or the presence of tumoral masses)

While moving the ultrasound probe along the dental arch of the patient, the method may be performed to detect any abnormalities of the detected anatomical structures, i.e. either abnormal periodontal pockets or the presence of a tumoral mass.

Then, during step 825 of the method 800, several predetermined features are then measured on the built images, such as the depths of the detected periodontal pockets, the high echogenicity indicator, the texture complexity and the texture inhomogeneity.

In case of multiple assessments during the same exam, the measured values will be displayed either all on the same page at the same time or successively one by one. Those displays will be directly on the LCD screen of the probe or on the acquisition interface of the computer of the remote workstation. Without any operator intervention, this display will last during a few seconds, for instance 5 seconds.

According to some embodiments, the measurements performed during the examination may be reported in a computer file. Such a computer file may be generated and stored in the acquisition workstation, either in the memory of the computer of the remote workstation, or the memory of the probe.

## Claims

1. A method (800) of detecting an abnormality along a portion of a dental arch using scanning material comprising an ultrasonic periodontal probe (300) configured for emitting ultrasound signals within at least one emitting cone and for receiving corresponding echoed ultrasound signals, the method comprising moving the probe (300) along a portion of the dental arch (500, 505), wherein while moving the probe (300) along the portion of the dental arch (500, 505), following steps are performed by the probe:
emitting an ultrasound signal within the emitting cone;
measuring echoed ultrasound signals;
detecting at least one anatomical structure to be investigated;
the probe comprising a microcontroller configured for
measuring at least one predetermined feature of the at least one detected anatomical structure to obtain a value of the measurement of the at least one predetermined feature characterizing the detected anatomical structure;
detecting an abnormality as a function of a threshold and the value of the measurement of the predetermined feature of the at least one detected anatomical structure,
the probe further comprising at least an indicator (425,455) configured to provide an item of information regarding an identified abnormality, the method further comprising energizing the indicator to indicate a detected abnormality.

2. The method according to claim 1, wherein detecting at least one anatomical structure to be investigated comprises:
building images continuously based on the measured echoed signals;
analyzing the built images in order to detect at least one anatomical structure.

3. The method according to claim 1 or 2, the physical quantity relating to the at least one detected anatomical structure is one of a dimension, an echogenicity indicator, a texture inhomogeneity, a texture complexity.

4. The method according to claim 2, wherein analyzing the built images comprises segmenting the built images for detecting at least one anatomical structure in the built images.

5. The method according to any one claim 1 to 4, wherein the at least one anatomical structure comprises alone or in combination a tooth and/or a gum and/or a periodontal pocket and/or a cortical bone.

6. The method according to claim 5, wherein the physical quantity relating to a detected periodontal pocket is a dimension such as depth.

7. The method according to claim 1 to 6, wherein each measurement is notified to the operator while moving the probe (300) along the portion of the dental arch (500, 505).

8. The method according to claim 1 to 7, wherein a maximal measurement value and/or a minimal measurement value is notified to the operator while moving the probe (300) along the portion of the dental arch (500, 505).

9. The method according to any one of claims 1 to 8, wherein emitting an ultrasound signal comprises recording an angulation of the probe (300) and/or recording the position of the probe (300).

10. The method according to any one of claims 1 to 9, wherein the ultrasonic periodontal probe (300) is configured for emitting ultrasound signals within at least one of several emitting cones extending in different directions.

11. The method according to claim 10, wherein emitting an ultrasound signal comprises selecting at least one of the several emitting cones and emitting an ultrasound signal within the at least one selected emitting cone.

12. The method according to any one of claims 1 to 11, wherein the indicator comprises one or more light emitting devices (330) and/or LCD screen and/or vibration means and/or sound effects.

## Patentansprüche

1. Verfahren (800) zum Detektieren einer Anomalie entlang eines Abschnitts eines Zahnbogens unter Verwendung von Scanmaterial, das eine Ultraschall-Parodontalsonde (300) umfasst, die zum Aussenden von Ultraschallsignalen innerhalb mindestens eines Sendekegels und zum Empfangen entsprechender zurückgeworfener Ultraschallsignale konfiguriert ist, wobei das Verfahren das Bewegen der Sonde (300) entlang eines Abschnitts des Zahnbogens (500, 505) umfasst, wobei während des Bewegens der Sonde (300) entlang des Abschnitts des Zahnbogens (500, 505) folgende Schritte von der Sonde durchgeführt werden:
Aussenden eines Ultraschallsignals innerhalb des Sendekegels;
Messen von zurückgeworfenen Ultraschallsignalen;
Detektieren mindestens einer zu untersuchenden anatomischen Struktur;
wobei die Sonde einen Mikrocontroller umfasst, der zum Messen mindestens eines vorbestimmten Merkmals der mindestens einen detektierten anatomischen Struktur, um einen Wert der Messung des mindestens einen vorbestimmten Merkmals zu erhalten, das die detektierte anatomische Struktur charakterisiert;
Detektieren einer Anomalie in Abhängigkeit von einem Schwellenwert und dem Wert der Messung des vorbestimmten Merkmals der mindestens einen detektierten anatomischen Struktur konfiguriert ist, wobei die Sonde ferner mindestens einen Indikator (425, 455) umfasst, der so konfiguriert ist, dass er eine Information über eine identifizierte Anomalie bereitstellt, wobei das Verfahren ferner das Aktivieren des Indikators umfasst, um eine detektierte Anomalie anzuzeigen.

2. Verfahren nach Anspruch 1, wobei das Detektieren mindestens einer zu untersuchenden anatomischen Struktur umfasst:
kontinuierliches Erstellen von Bildern auf der Grundlage der gemessenen zurückgeworfenen Signale;
Analysieren der erstellten Bilder, um mindestens eine anatomische Struktur zu detektieren.

3. Verfahren nach Anspruch 1 oder 2, wobei die physikalische Größe, die sich auf die mindestens eine detektierte anatomische Struktur bezieht, eine Dimension, einen Echogenitätsindikator, eine Texturinhomogenität oder eine Texturkomplexität ist.

4. Verfahren nach Anspruch 2, wobei das Analysieren der erstellten Bilder das Segmentieren der erstellten Bilder zum Detektieren mindestens einer anatomischen Struktur in den erstellten Bildern umfasst.

5. Verfahren nach einem der Ansprüche 1 bis 4, wobei die mindestens eine anatomische Struktur allein oder in Kombination einen Zahn und/oder ein Zahnfleisch und/oder eine Parodontaltasche und/oder einen kortikalen Knochen umfasst.

6. Verfahren nach Anspruch 5, wobei die physikalische Größe, die sich auf eine detektierte Parodontaltasche bezieht, eine Dimension wie Tiefe ist.

7. Verfahren nach Anspruch 1 bis 6, wobei jede Messung dem Bediener mitgeteilt wird, während die Sonde (300) entlang des Abschnitts des Zahnbogens (500, 505) bewegt wird.

8. Verfahren nach Anspruch 1 bis 7, wobei dem Bediener ein maximaler Messwert und/oder ein minimaler Messwert mitgeteilt wird, während die Sonde (300) entlang des Abschnitts des Zahnbogens (500, 505) bewegt wird.

9. Verfahren nach einem der Ansprüche 1 bis 8, wobei das Aussenden eines Ultraschallsignals das Aufzeichnen einer Abwinkelung der Sonde (300) und/oder das Aufzeichnen der Position der Sonde (300) umfasst.

10. Verfahren nach einem der Ansprüche 1 bis 9, wobei die Ultraschall-Parodontalsonde (300) so konfiguriert ist, dass sie Ultraschallsignale innerhalb von mindestens einem von mehreren, sich in verschiedene Richtungen erstreckenden Sendekegeln aussendet.

11. Verfahren nach Anspruch 10, wobei das Aussenden eines Ultraschallsignals das Auswählen von mindestens einem der mehreren Sendekegel und das Aussenden eines Ultraschallsignals innerhalb des mindestens einen ausgewählten Sendekegels umfasst.

12. Verfahren nach einem der Ansprüche 1 bis 11, wobei der Indikator eine oder mehrere lichtemittierende Vorrichtungen (330) und/oder einen LCD-Bildschirm und/oder Vibrationsmittel und/oder Klangeffekte umfasst.

## Revendications

1. Procédé (800) de détection d'une anomalie le long d'une partie d'arcade dentaire à l'aide d'un matériel de balayage comprenant une sonde parodontale ultrasonore (300) configurée pour émettre des signaux ultrasonores dans au moins un cône d'émission et pour recevoir les signaux ultrasonores réfléchis correspondants, le procédé comprenant le déplacement de la sonde (300) le long d'une partie de l'arcade dentaire (500, 505), dans lequel, pendant le déplacement de la sonde (300) le long de la partie de l'arcade dentaire (500, 505), les étapes suivantes sont réalisées par la sonde :
l'émission d'un signal ultrasonore à l'intérieur du cône d'émission ;
la mesure des signaux ultrasonores réfléchis ;
la détection d'au moins une structure anatomique à examiner ;
la sonde comprenant un microcontrôleur configuré pour mesurer au moins une caractéristique prédéterminée de l'au moins une structure anatomique détectée afin d'obtenir une valeur de la mesure de l'au moins une caractéristique prédéterminée caractérisant la structure anatomique détectée ;
détecter une anomalie en fonction d'un seuil et de la valeur de la mesure de la caractéristique prédéterminée de l'au moins une structure anatomique détectée, la sonde comprenant en outre au moins un indicateur (425, 455) configuré pour fournir une information concernant une anomalie identifiée, le procédé comprenant en outre l'excitation de l'indicateur pour signaler une anomalie détectée.

2. Procédé selon la revendication 1, dans lequel la détection d'au moins une structure anatomique à examiner comprend :
la construction d'images en continu à partir des signaux réfléchis mesurés ;
l'analyse des images construites afin de détecter au moins une structure anatomique.

3. Procédé selon la revendication 1 ou 2, dans lequel la quantité physique relative à l'au moins une structure anatomique détectée est l'une d'une dimension, d'un indicateur d'échogénicité, d'une inhomogénéité de texture, d'une complexité de texture.

4. Procédé selon la revendication 2, dans lequel l'analyse des images construites comprend la segmentation des images construites afin de détecter au moins une structure anatomique dans les images construites.

5. Procédé selon l'une quelconque des revendications 1 à 4, dans lequel l'au moins une structure anatomique comprend, seule ou en combinaison, une dent et/ou une gencive et/ou une poche parodontale et/ou un os cortical.

6. Procédé selon la revendication 5, dans lequel la quantité physique relative à une poche parodontale détectée est une dimension telle que la profondeur.

7. Procédé selon la revendication 1 à 6, dans lequel chaque mesure est communiquée à l'opérateur lors du déplacement de la sonde (300) le long de la partie de l'arcade dentaire (500, 505).

8. Procédé selon la revendication 1 à 7, dans lequel une valeur de mesure maximale et/ou une valeur de mesure minimale est communiquée à l'opérateur lors du déplacement de la sonde (300) le long de la partie de l'arcade dentaire (500, 505).

9. Procédé selon l'une quelconque des revendications 1 à 8, dans lequel l'émission d'un signal ultrasonore comprend l'enregistrement d'une angulation de la sonde (300) et/ou l'enregistrement de la position de la sonde (300).

10. Procédé selon l'une quelconque des revendications 1 à 9, dans lequel la sonde parodontale ultrasonore (300) est configurée pour émettre des signaux ultrasonores dans au moins l'un de plusieurs cônes d'émission s'étendant dans des directions différentes.

11. Procédé selon la revendication 10, dans lequel l'émission d'un signal ultrasonore consiste à sélectionner au moins l'un des plusieurs cônes d'émission et à émettre un signal ultrasonore dans l'au moins un cône d'émission sélectionné.

12. Procédé selon l'une quelconque des revendications 1 à 11, dans lequel l'indicateur comprend un ou plusieurs dispositifs électroluminescents (330) et/ou un écran LCD et/ou des moyens de vibration et/ou des effets sonores.
